(19) **Europäisches Patentamt / European Patent Office / Office européen des brevets**

(11) **EP 3 150 659 A1**

(12) **EUROPEAN PATENT APPLICATION**
published in accordance with Art. 153(4) EPC

(43) Date of publication:
**05.04.2017 Bulletin 2017/14**

(21) Application number: **15799921.0**

(22) Date of filing: **26.05.2015**

(51) Int Cl.:
*C08J 11/28* (2006.01)   *C09K 8/12* (2006.01)
*C12N 9/18* (2006.01)   *E21B 43/22* (2006.01)

(86) International application number:
**PCT/JP2015/065143**

(87) International publication number:
**WO 2015/182622 (03.12.2015 Gazette 2015/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **26.05.2014 JP 2014107876**

(71) Applicant: **Toyo Seikan Group Holdings, Ltd.**
**Shinagawa-ku**
**Tokyo 141-8627 (JP)**

(72) Inventors:
• **KATAYAMA, Tsutaki**
  **Yokohama-shi**
  **Kanagawa 240-0062 (JP)**
• **YOSHIKAWA, Seishi**
  **Yokohama-shi**
  **Kanagawa 240-0062 (JP)**

(74) Representative: **Raynor, Stuart Andrew**
  **J A Kemp**
  **14 South Square**
  **Gray's Inn**
  **London WC1R 5JJ (GB)**

(54) **METHOD FOR DECOMPOSING ESTER RESIN**

(57)   A method of decomposing an ester resin with an enzyme by making present an ester decomposition promotor, an enzyme and an enzyme adsorption-suppressing compound in an aqueous medium that contains the ester resin. The decomposition method is capable of decomposing, at a low temperature maintaining reliability and stability, the ester resin added to a dispersion solution for drilling, such as fracturing fluid.

Fig. 1

NaCl 3%

**Description**

Technical Field:

This invention relates to a method of decomposing ester resins with an enzyme. More specifically, the invention relates to a method of decomposing, with an enzyme, ester resins that are added into a dispersion solution for drilling that is used for extracting underground resources by an ore chute drilling method.

Background Art:

Ore chute drilling methods such as hydraulic fracturing method, rotary drilling method and riserless drilling method have now been widely employed for extracting the underground resources.

The rotary drilling method consists of forming the ore chute by drilling while refluxing the mud and forming a filter cake called mud wall on the wall surfaces of the ore chute using a finishing fluid blended with a water loss-preventing agent. The cake maintains the chute walls stable, prevents the chute walls from collapsing and reduces friction to the fluid flowing through the ore chute.

The hydraulic fracturing method consists of pressurizing the fluid filled in the ore chute to form cracks (fractures) in the vicinities of the ore chute to thereby improve permeability in the vicinities of the ore chute (for easy flow of the fluid) in order to increase the effective sectional area through which the resources such as oils and gases flow into the ore chute and, therefore, to improve productivity of the ore chute.

Here, as the water loss-preventing agent that is added to the finishing fluid, there are chiefly used calcium carbonate or various kinds of salts in a granular form. However, use of the water loss-preventing agent brings about such problems that it becomes necessary to conduct a treatment with acid to remove it, or the water loss-preventing agent stays clogged in the stratum from where the resources are to be extracted hindering the production.

Further, the fluid used in the hydraulic fracturing method is also called fracturing fluid. So far, a viscous fluid like jelly gasoline was used. However, as the shale gas or the like gas has now been extracted from the shale layer that exists in relatively shallow places and by taking the effects on the environment into consideration, it is becoming a common practice to use an aqueous dispersion solution obtained by dissolving or dispersing a polymer in water. As such a polymer, there has been known a polylactic acid (see a patent document 1).

The polylactic acid is a substance that exhibits hydrolysable capability and biodegradable capability, and, even if it remains under the ground, is decomposed with water, enzyme or microorganisms in the ground and does not adversely affect the environment. Further, the water that is used as a dispersant, too, can be considered to be far from affecting the environment as compared to gasoline or the like.

Further, the ore chute is filled with the aqueous dispersion solution in which the polylactic acid is dispersed, and the aqueous dispersion solution is pressurized so that the polylactic acid permeates into the vicinities of the ore chute. Here, the polylactic acid undergoes the hydrolysis and loses the form of the resin. Therefore, spaces (or cracks) form in the portions where the polylactic acid aqueous dispersion has permeated accounting for an increase in space of the ore chute into which the resources can flow.

The polylactic acid, further, works as a water loss-preventing agent and suppresses the water used as the dispersion medium from permeating into the ground too much. Therefore, the polylactic acid offers an advantage of minimizing a change in the environment in the stratum. Besides, no treatment with acid is necessary since it decomposes in the ground.

In addition, after the polylactic acid is decomposed, a lactic acid is released, the lactic acid being a decomposed product of the polylactic acid and being a kind of organic acid. The lactic acid corrodes the shale layer and, as a result, accelerates the shale layer to become porous.

However, though the polylactic acid undergoes the hydrolysis relatively quickly at high temperatures, its rate of hydrolysis becomes small as the temperature becomes low. Therefore, the production efficiency is poor if it is used for extracting the shale gases and the like gases from the ground where the temperature is low, and improvements have been desired.

On the other hand, there has been proposed the use of a polyglycolic acid instead of the polylactic acid (see a patent document 2).

The polyglycolic acid, too, has been known as a biodegradable resin. The polyglycolic acid is more highly hydrolysable than the polylactic acid. For instance, at a temperature of about 80°C, it undergoes the hydrolysis at a rate considerably higher than that of the polylactic acid. Therefore, the polyglycolic acid can be effectively used to substitute for the polylactic acid.

The problem, however, is that the polyglycolic acid is considerably expensive as compared to the polylactic acid. The polyglycolic acid which is expensive causes a fatal problem if it is to be used for the hydraulic fracturing method that uses a fracturing fluid in large amounts. Besides, the polyglycolic acid involves another problem in that it cannot be decomposed to a satisfactory degree under specific temperature conditions.

Under such circumstances, the present applicant has previously proposed a dispersion solution for drilling obtained by positively adding an enzyme to an ester resin that can be decomposed with an enzyme (see a patent document 3) . Concretely speaking, the patent document 3 discloses an art of adding an enzyme, in advance, to a dispersion solution that contains an enzymatically decomposable ester resin, such as polylactic acid. Therefore, the dispersion solution disclosed in the patent document 3 causes the resin to be highly decomposed and accelerates the decomposition of the polylactic acid and the like at low temperatures.

The enzyme-containing dispersion solution for drilling taught in the patent document 3 exhibits excellent decomposing capability in the drilling for recovering resources if tested on a laboratory scale. In the practical use, however, the present inventors have discovered that the dispersion solution fails to exhibit its excellent decomposing capability as tested on a laboratory scale and, therefore, that the decomposing capability must be further improved.

Prior Art Documents:

Patent Documents:

Patent document 1: USP 7,833,950
Patent document 2: WO 2012/050187
Patent document 3: WO 2014/092146

Outline of the Invention:

Problems that the Invention is to Solve:

It is, therefore, an object of the present invention to provide a decomposition method capable of decomposing, at a low temperature maintaining reliability and stability, an ester resin added to a dispersion solution for drilling, such as fracturing fluid.

Another object of the present invention is to provide an aqueous dispersion solution that can be decomposed with an enzyme and can be used for extracting the underground resources, and to a method of extracting the underground resources by using the dispersion solution.

Means for Solving the Problems:

According to the present invention, there is provided a method of decomposing an ester resin with an enzyme by making present an ester decomposition promotor, an enzyme and an enzyme adsorption-suppressing compound in an aqueous medium that contains the ester resin.

In the decomposition method of the present invention, it is desired to employ the following means:

(1) The ester resin is a polylactic acid;
(2) As the ester decomposition promoter, use is made of an acid-releasing hydrolysable resin that releases an oxalic acid or a glycolic acid upon the hydrolysis;
(3) The acid-releasing hydrolysable resin is a polyoxalate;
(4) As the enzyme, use is made of at least the one selected from protease, lipase and cutinase; and
(5) A trishydroxymethylaminomethane is used as the enzyme adsorption-suppressing compound.

According to the present invention, further, there is provided an enzymatically decomposable aqueous dispersion solution comprising an aqueous medium in which are dispersed an ester resin, an ester decomposition promoter for promoting the hydrolysis of the ester resin, and an enzyme, and in which is, further, contained an enzyme adsorption-suppressing compound.

In the enzymatically decomposable aqueous dispersion solution of the invention, it is desired that the enzyme adsorption-suppressing compound is contained in amount of not less than 0.01% by mass relative to the aqueous medium.

The enzymatically decomposable aqueous dispersion solution is used, specifically, as a dispersion solution for drilling the underground resources.

According to the present invention, further, there is provided a method of extracting the underground resources from an ore chute formed by drilling, including the step of hydrolyzing an ester resin in water of not lower than 40°C by forcibly introducing the enzymatically decomposable aqueous dispersion solution into the ore chute.

Effects of the Invention:

The present invention has a remarkable feature in that an aqueous medium that contains an enzymatically decomposable ester resin as represented by a polylactic acid is, further, added with an enzyme that decomposes the ester resin, an ester decomposition promoter and an enzyme adsorption-suppressing compound. Owing to this feature, the present invention makes it possible to reliably and quickly decompose the ester resin.

That is, upon adding the enzyme into the aqueous medium that contains the enzymatically decomposable ester resin, decomposition of the ester resin is promoted. Here, if a salt such as sodium chloride is mixed in the aqueous medium, the enzymatic activity decreases and the ester resin decomposes at a greatly decreased rate. The dispersion solution for drilling used for drilling the underground resources uses large amounts of water as the aqueous medium. Here, however, salts such as sodium chloride and the like gradually emerge from the ground and dissolve therein; i.e., the salt concentration gradually increases. Further, the underground resources may often be extracted from the bottom of the sea. In such a case, the sea water is used as the aqueous medium, and the salt concentration is as high as 3% by weight or more from the first time. Namely, in case the aqueous dispersion solution containing the ester resin and the enzyme is used as the dispersion solution for extraction, the enzymatic activity decreases due to an increase in the concentration of salts such as sodium chloride and the like. As a result, it is considered that the decomposition of the ester resin can no longer be promoted maintaining stability.

According to the present invention, on the other hand, the enzyme adsorption-suppressing compound is made present together with the ester decomposition promoter. This makes it possible to effectively avoid a decrease in the rate of decomposing the ester resin caused by the infiltration of salts and to secure excellent decomposing capability maintaining reliability.

Therefore, the decomposition method of the present invention can be effectively utilized under the circumstances where salts may infiltrate, and can be effectively adopted to extracting, specifically, the underground resources. For instance, an aqueous dispersion solution containing the ester resin, enzyme, ester decomposition promoter and enzyme adsorption-suppressing compound, can be effectively used as a dispersion solution for drilling the underground resources.

Brief Description of the Drawings:

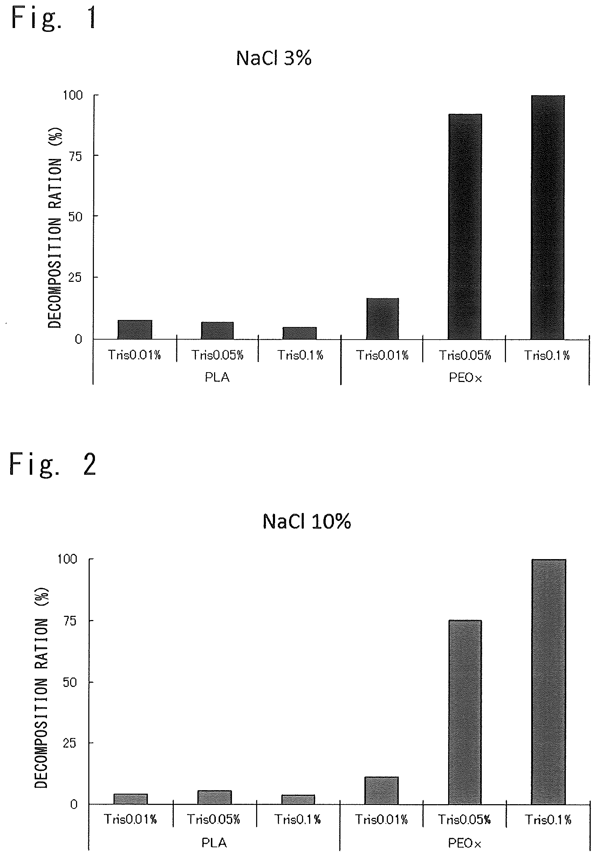

[Fig. 1] is a graph showing decomposition ratios at an NaCl concentration of 3% in Examples of the invention.
[Fig. 2] is a graph showing decomposition ratios at an NaCl concentration of 10% in Examples of the invention.

Modes for Carrying Out the Invention:

<Ester Resin>

There is no specific limitation on the ester resin that is used in the present invention provided it can be decomposed with an enzyme. When the present invention is used for drilling the underground resources, however, there is used an ester resin which is insoluble in water. If soluble in water, the ester resin permeates into the ground too much and affects the environment to a large degree. Specifically, if soluble in water, then the ester resin that is contained as a component in the fracturing fluid cannot cause the ground to be cracked and cannot, either, serve as a filler; i.e., the ester resin is quite useless.

As the water-insoluble ester resin that can be decomposed with the enzyme and that can be used for drilling underground resources, there can be exemplified polylactic acid, polyhydroxyalkanoate, polycaprolactone, polybutylene succinate, polybutylene succinate adipate, polybutylene terephthalate adipate, cellulose acetate and thermoplastic starch. They also can be used in the form of a blend thereof.

Specifically, the polylactic acid is best suited as the ester resin. This is because upon being used in combination with an enzyme described later, the polylactic acid maintains a suitable degree of stability (e.g., a mass-holding ratio of not less than 80% at a temperature of 45°C after a holding time of 3 hours) and a high decomposition ratio at a temperature of not higher than 50°C (e.g., a mass-holding ratio of not more than 50% at a temperature of 45°C after a holding time of 96 hours and, further, a mass-holding ratio of not more than 20% after a holding time of 168 hours). Besides, the polylactic acid is inexpensive and is suited for the use of drilling the underground resources.

The polylactic acid may be either a 100% of poly-L-lactic acid or a 100% of poly-D-lactic acid, or may be a molten blend of the poly-L-lactic acid and the poly-D-lactic acid, or may be a random copolymer or a block copolymer of the L-lactic acid and the D-lactic acid.

The above enzymatically decomposable resin can be used also in the form of a copolymer being copolymerized with various kinds of aliphatic polyhydric alcohols, aliphatic polybasic acid, hydroxycarboxylic acid or lactone so far as the above-mentioned stability and decomposability are not spoiled.

As the polyhydric alcohols, there can be exemplified ethylene glycol, propylene glycol, butanediol, octanediol, dodecanediol, neopentyl glycol, glycerin, pentaerythritol, sorbitan and polyethylene glycol.

As the polybasic acid, there can be exemplified succinic acid, adipic acid, sebacic acid, glutaric acid, decanedicarboxylic acid, cyclohexanedicarboxylic acid and terephthalic acid. It is also allowable to use a carboxylic acid diester.

As the hydroxycarboxylic acid, there can be exemplified glycolic acid, hydroxypropionic acid, hydroxyvaleric acid, hydroxycaproic acid and mandelic acid.

As the lactone, there can be exemplified caprolactone, butylolactone, valerolactone, propiolactone, undecalactone, glycolide and mandelide.

As required, further, there can be added such additives as known plasticizer, heat stabilizer, photo stabilizer, antioxidant, ultraviolet ray absorber, flame retardant, coloring agent, pigment, filler, parting agent, antistatic agent, perfume, lubricant, foaming agent, antibacterial antifungal agent and nucleating agent in their own forms or being dispersed in water.

When used as the fracturing fluid, the above-mentioned ester resin should have a suitable degree of molecular weight so as to work as a filler yet permeating into the ground. Usually, therefore, the ester resin should have a weight average molecular weight in a range of 5,000 to 1,000,000 and, specifically, 10,000 to 500,000.

Further, by using a forming means known per se. , the ester resin is formed into pellets, a granular material like powder, film, or pulverized product obtained by pulverizing the films, or into a fiber of a single layer or of a core-sheath structure, or into capsules, which are then dispersed in water.

<Ester decomposition promoter>

In the present invention, the ester decomposition promoter is used for promoting the hydrolysis of the above-mentioned ester resin. The ester decomposition promoter by itself does not work to decompose the ester. When mixed with water, however, it releases an acid or an alkali that works as a catalyst for decomposing the ester.

Usually, the ester decomposition promoter is homogeneously dispersed in a formed body (film, fiber, etc.) of the ester resin. Desirably, the ester decomposition promoter has a weight average molecular weight of, for example, 1, 000 to 200, 000 from the standpoint of quickly hydrolyzing the resin with the acid or the alkali that is released.

Of the ester decomposition promoters, the one that releases alkali may comprise an alkali metal salt of acrylic acid such as sodium acrylate or sodium alginate. However, the one that releases an alkali is prone to adversely affect the environment. Usually, therefore, the ester decomposition promotor that releases an acid is favorably used.

Concretely speaking, when dissolved or dispersed in an aqueous solution at a concentration of 0.005 g/ml of water, the acid-releasing ester decomposition promoter exhibits a pH (25°C) of not more than 4 and, specifically, not more than 3. A polymer is, preferably, used since it easily undergoes the hydrolysis and releases an acid when it is mixed with water.

As the polymer, there can be exemplified acid-releasing hydrolysable resins such as polyoxalate and polyglycolic acid which, upon being hydrolyzed, release an oxalic acid or a glycolic acid. They may be used as copolymers, or may be used in a single kind or in a combination of two or more kinds.

As the components for forming copolymers, there can be exemplified:

polyhydric alcohols such as ethylene glycol, propylene glycol, butanediol, octanediol, dodecanediol, neopentyl glycol, glycerin, pentaerythritol, sorbitan, bisphenol A and polyethylene glycol;
dicarboxylic acids such as succinic acid, adipic acid, sebacic acid, glutaric acid, decanedicarboxylic acid, cyclohexanedicarboylic acid, terephthalic acid isophthalic acid and anthracendicarboxylic acid;
hydroxycarboxylic acids such as glycolic acid, L-lactic acid, D-lactic acid, hydroxypropionic acid, hydroxybutyric acid, hydroxyvaleric acid, hydroxycaproic acid, mandelic acid and hydroxybenzoic acid; and
lactones such as glycolide, caprolactone, butylolactone, valerolactone, propiolactone and undecalactone.

In the specification, the polyoxalate stands for a homopolymer, a copolymer or a blend thereof in which a polymer is formed by polymerizing at least the oxalic acid as a monomer.

Specifically, the polyoxalate and the polyglycolic acid are easily hydrolysable resins, quickly undergo the hydrolysis, and work to excellently promote the hydrolysis of the sparingly hydrolysable ester resins. Among them, the polyoxalate and, specifically, the polyethylene oxalate works to very highly promote the hydrolysis as compared to the polyglycolic acid, i.e., works to very highly promote the hydrolysis of the polylactic acid and the like that are sparingly hydrolysable even at temperatures of not higher than 80°C. Besides, they are very less expensive than the polyglycolic acid, and offer a great advantage in cost.

The above ester decomposition promoter is, usually, used in an amount of 1 to 80 parts by mass and, specifically, 5 to 70 parts by mass per 100 parts by mass of the ester resin. If the amount thereof is too small, decomposition of the ester resin is not promoted to a satisfactory degree. If the amount thereof is unnecessarily large, on the other hand, a disadvantage in cost becomes more conspicuous than the advantage of promoting the decomposition of the ester resin. Besides, depending on the cases, the ester resin is decomposed to such an excess degree that it becomes difficult to handle the hydrolysis or it becomes difficult to effectively utilize the ester resin.

<Enzyme>

The present invention uses an enzyme together with the ester decomposition promoter, the enzyme working to secure a high decomposition capability at low temperatures (not higher than 50°C). For example, by using a solution blended with no enzyme but in which the ester resin is dispersed, the mass-holding ratio becomes far greater than 50% after held at 45°C for 96 hours (4 days), and a high decomposition capability is not realized.

A suitable enzyme is used depending on the kind of the ester resin that is used. Specifically, when a polylactic acid is used, there is desirably used protease, cellulose, cutinase, lipase, esterase, chitinase, xylanase and PHB depolymerase. Specifically, it is most desired to use at least any one of protease, cutinase and lipase.

These enzymes may have been fixed or may not have been fixed. For instance, the Protenase K or the like manufactured by Wako-Junyaku Co. is used in the form an aqueous solution. Further, exoenzyme may be used by putting microorganisms to it. In this case, there may be added a culture component or a nutrient component required for the microorganisms.

The above-mentioned enzyme is used in an amount of, usually, 10 to 200 parts by weight and, specifically, 25 to 100 parts by weight per 100 parts by weight of the ester resin, though the amount of the enzyme may vary depending on its kind.

< Enzyme adsorption-suppressing compound >

In the present invention, an enzyme adsorption-suppressing compound is made present at the time of decomposing the ester resin in an aqueous medium by using the above-mentioned ester decomposition promoter and the enzyme.

The enzyme adsorption-suppressing compound acts on the ester resin-adsorbing domains of the enzyme to suppress the ester resin from adsorbing the enzyme. A decrease in the amount of enzyme adsorption can be confirmed by, for example, a method described below. That is, a film of the ester resin is dipped in an aqueous solution containing 0.5% by mass of Tris and a predetermined amount of NaCl. While maintaining the aqueous solution at 0°C, the enzyme is added to the aqueous solution with stirring. While maintaining the above temperature, the enzyme adsorption-suppressing compound acts on the ester resin-adsorbing domains of the enzyme and causes the amount of enzyme adsorption to decrease. The amount of enzyme adsorption can be measured by the QCM (crystal oscillator microbalance).

In the present invention, it is, usually, desired that after the enzyme adsorption-suppressing compound is added at 0°C, the amount of enzyme adsorption is decreased by not less than 20% and, specifically, by not less than 50% after 5 minutes though it may vary depending on the measuring conditions.

As the enzyme adsorption-suppressing compound, there can be exemplified:

trishydroxymethylaminomethane (Tris) and hydrochlorides thereof;
N-cyclohexyl-2-aminoethanesulfonic acid (CHES);
N,N-bis(2-hydroxyethyl)glycine (Bicine);
N-[tris(hydroxymethyl)methyl]-3-aminopropanesulfonic acid (TAPS);
N-(2-hydroxy-1,1-bis(hydroxymethyl)ethyl)glycine (Tricine); and the like.

By using the phosphate aqueous solution that has generally been known as a so-called buffer solution like Tris, however, the phosphoric acid or a salt thereof does not act on the ester resin-adsorbing domains of the enzyme. Therefore, the above solution cannot be used in the present invention.

In the present invention, the enzyme adsorption-suppressing compound is used, desirably, in an amount of not less than 0.01% by mass, specifically, not less than 0.05% by mass and, more desirably, in an amount of 0.05 to 5% by mass relative to the aqueous medium so as to effectively exhibit its capability for enzyme adsorption-suppressing without lowering the enzymatic activity.

<Decomposition of ester resin>

In the invention, the ester resin is dispersed in the aqueous medium (usually, water) together with the ester decomposition promoter, enzyme and enzyme adsorption-suppressing compound in an amount as descried above, and is decomposed.

The amount of the aqueous medium that is used is such that the ester resin and the ester decomposition promoter are effectively hydrolyzed.

In the decomposition method of the present invention, the hydrolysis of the ester resin is promoted by an acid or an alkali released by the hydrolysis of the ester decomposition promoter and, at the same time, the ester resin is decomposed by the enzymatic reaction. Here, the enzyme adsorption-suppressing compound is present in the reaction system. Therefore, the enzyme is effectively suppressed from being adsorbed by the ester resin and the decomposition reaction with the enzyme is carried out maintaining stability. That is, even if a salt such as sodium chloride is mixed into the reaction system, the enzymatic reaction is effectively prevented from being impaired, enabling the ester resin to be effectively decomposed by the enzymatic reaction in an accelerated manner.

Moreover, the enzyme also promotes the hydrolysis of the ester decomposition promoter. Accordingly, the decomposition of the ester resin is more effectively promoted.

For example, as demonstrated in Examples appearing later, when the enzyme adsorption-suppressing compound (Tris) is used in an amount of 0.1% by mass relative to the aqueous medium, the ester resin can be all decomposed irrespective of if NaCl is mixed in an amount of 3% or 10%. On the other hand, when the enzyme adsorption-suppressing compound is not used, the ester resin is decomposed in an amount of less than 5% even if NaCl is mixed in an amount of about 3%.

According to the decomposition method of the present invention as will be understood from the above description, the decomposition of the ester resin can be effectively promoted despite the NaCl concentration is not less than 3%. This is a great advantage of the present invention making it possible to use the seawater (NaCl concentration of about 3 to about 4%) as the aqueous medium.

<Dispersion solution for drilling>

The above-mentioned method of decomposing the ester resin with the enzyme of the present invention can be effectively adopted to a dispersion solution for drilling that contains, as an effective component, the enzymatically decomposable ester resin as represented by the polyactic acid in the form of a formed body such as the film mentioned earlier.

The dispersion solution is thrown into the ore chute drilled in the ground, and is utilized for extracting the underground resources, for example, for collecting shale gases from the shale layer relying on the ore chute drilling such as rotary drilling or hydraulic fracturing. The ester resin is, usually, added to the aqueous dispersion solution in an amount of 0.01 to 20% by mass and, specifically, 0.01 to 10% by mass. Into the above dispersion solution, there are added the ester decomposition promoter, the enzyme and the enzyme adsorption-suppressing compound.

The above dispersion solution for drilling can be, further, blended with a water-absorbing polymer such as polyvinyl alcohol or CMC in addition to the components mentioned above. Upon being blended with the water-soluble polymer, it is allowed to suppress the hydrolysis of the ester resin before the hydraulic fracturing is carried out and to improve easiness for handling the fracturing fluid.

If the water-absorbing polymer is used in too large amounts, however, it becomes probable that the enzymatically decomposable resin loses its function. Usually, therefore, the water-absorbing polymer is used in an amount of not more than 50 parts by mass and, specifically, in a range of 1 to 10 parts by mass per 100 parts by mass of the ester resin.

The dispersion solution for drilling can be, further, blended with known additives that are used for the ore chute drilling such as hydraulic fracturing and the rotary drilling.

In the case of the hydraulic fracturing, for example, there is added a proppant that contains a water-soluble polysaccharide (gelling agent) such as guar gum as a thickener and sand (support agent) so that the cracks formed by the hydraulic fracturing will not be clogged. Here, the water-soluble polysaccharide such as guar gum may consume the enzyme and may lose the activity. In the invention, however, it has been confirmed that the enzyme is consumed predominantly in the hydrolysis of the ester resin and does not adversely affect the hydrolysis of the ester resin.

It is, further, allowable to add a surfactant for dispersing the ester resin.

It is desired that any of the above additives are added to the aqueous dispersion solution in which the ester resin and the enzyme are dispersed in amounts as described above, the additives being added in such amounts as will not impair the function of the ester resin or the decomposability of the ester resin.

<Drilling by the hydraulic fracturing>

The above-mentioned dispersion solution for drilling is introduced with pressure into the ground where the ester resin in the dispersion solution is hydrolyzed with the enzyme at a temperature of not lower than 40°C (specifically, at 40 to 50°C). Therefore, the dispersion solution for drilling is very useful as a finishing solution used in drilling the ore chute or as a fracturing fluid used for extracting the underground resources by the hydraulic fracturing method.

Concretely, a vertical shaft is drilled down to the stratum where the desired underground resources are present.

Next, a horizontal hole is drilled in a horizontal direction to form the ore chute.

The thus formed ore chute is filled with the dispersion solution for drilling that, as required, contains the proppants such as gelling agent and support agent, and is pressurized so as to be fractured. Due to the pressure, the ester resin and the proppants infiltrate into the vicinities of the horizontal hole; i.e., the enzymatically decomposable resin material decomposes and extinguishes, and a pillar structure is formed. The remaining dispersion solution is sucked and, thereafter, the operation is commenced to recover the underground resources such as gases and oils.

If the hydraulic fracturing is carried out by using the above dispersion solution for drilling as the fracturing fluid, the ester resin remains stable to a suitable degree yet, at the same time, quickly undergoes the decomposition at temperatures of not higher than 50°C in short periods of time. Namely, the hydraulic fracturing is carried out efficiently. In addition to being used as the fracturing fluid, the dispersion solution for drilling can also be used as a plugging material or a breakdown material.

Further, when the ore chute is drilled while refluxing the mud, the dispersion solution for drilling can be used as an agent for adjusting the dehydration of the finishing fluid. This eliminates the need of conducting the treatment with an acid in a subsequent step, and the probability of clogging and trouble in the production.

Even if the resin permeates into unnecessarily wide regions and remains without being hydrolyzed, there is no probability of adversely affecting the environment.

Use of the dispersion solution for drilling causes the ester resin, ester decomposition promoter, enzyme and enzyme adsorption-suppressing compound to forcibly permeate into the ground together with other additives in a form being dispersed in water. Here, the enzyme and the enzyme adsorption-suppressing compound can be added in a subsequent step. For instance, after having introduced the liquid, in which the components other than the enzyme are dispersed, into the ore chute with pressure, an aqueous solution of the enzyme may be fed.

The water used as the dispersion medium may be the seawater which, however, is subject to be heated at a temperature in the ground depending on the position in the ore chute. Therefore, the water used as the dispersion medium may have been mixed with various dispersion components at room temperature, or may be mixed with other dispersion components in the form of hot water heated in advance at not lower than 40°C (usually, not higher than 50°C so that the enzyme will not lose its activity).

Further, enzyme adsorption-suppressing compound may be added after the concentration of NaCl has become not less than 3% by mass in the dispersion solution.

Moreover, even in case the temperature in the ground has become higher than 50°C at which the enzyme loses its activity, the hydrolysis of the ester resin is promoted by the ester decomposition promoter that is added to the dispersion solution minimizing the effect caused by a rise in the temperature.

EXAMPLES

The invention will now be described by way of Examples.

In Examples, various measurements were taken by the methods described below.

<Measuring the melting points and glass transition points>

Apparatus: DSC6220 (differential scanning calorimeter) manufactured by Seiko Instruments Co.
Sample preparation: amount of sample, 5 to 10 mg
Measuring conditions: in a nitrogen atmosphere, temperature rising rate of 10°C/min., temperature range of 0 to 250°C

<Measuring the molecular weights>

Apparatus: Gel permeation chromatograph GPC
Detector: Differential refractometer RI (Model RI-2414, sensitivity: 512, manufactured by Waters Co.)
Column: Shodex, HFIP-LG (one unit), HFIP-806M (2 units), manufactured by Showa Denko Co.
Solvent: Hexafluoroisopropanol (5 mM to which sodium trifluoroacetate is added)
Flow rate: 0.5 mL/min.
Column temperature; 40°C
Sample preparation: To about 1.5 mg of a sample, 5 mL of solvent was added, and the mixture was mildly stirred at room temperature (sample concentration of about 0.03%). After having confirmed with the eye that the sample has been dissolved, the mixture was passed through a 0.45-$\mu$m filter (repeated twice from the weighing). All samples were measured for their molecular weights within about one hour from the start of preparation.

<Synthesis of the ester decomposition promoter>

As the ester decomposition promoter, a polyethylene oxalate (hereinafter "PEOx") was synthesized by a method described below.

Into a one-liter separable flask equipped with a mantle heater, a stirrer, a nitrogen introduction pipe and a cooling pipe, there were introduced:

dimethyl oxalate, 472 g (4 mols),
ethylene glycol, 297 g (4.8 mols), and
tetrabutyl titanate, 0.42 g,

and the mixture was reacted for 7 hours in a nitrogen atmosphere by elevating the temperature in the flask from 120°C up to 180°C while distilling off methanol. Finally, 270 ml of methanol was distilled off.

Thereafter, the temperature in the flask was elevated to 170 to 190°C stepwise, the reaction was continued for 7 hours under a reduced pressure of 0.1 to 0.2 kPa, and the reaction product was taken out.

The polymer that was taken out was granulated by using a crusher, and was dried in vacuum at 110°C for 4 hours so as to be crystallized.

The obtained polymer possessed a weight average molecular weight of 70, 000, a melting point of 180°C and a glass transition temperature of 35°C.

<Preparation of PLA pellets containing the ester decomposition promoter>

A polylacetic acid (PLA: 4032D manufactured by Natureworks Co.) was dry-blended with the PEOx. By using a biaxial extruder (ULT Nano 05-20AG manufactured by Technovel Co.), the blend was melted and mixed at 200°C, and from which master pellets were prepared. The thus prepared master pellets were used as PLA pellets containing the ester decomposition promoter.

<Examples 1 to 6 and Comparative Examples 1 to 6>

Described below are the polylactic acid films, various agents and the procedures of experiments employed in Examples 1 to 6 and Comparative Examples 1 to 6.

Films;

In Examples 1 to 6, by using the Laboplusto-mill (manufactured by Toyo Seiki Co.), the PLA pellets containing the ester decomposition promoter were formed at 210°C into 100 $\mu$m-thick PLA films containing the ester decomposition promoter. Contents (% by weight) of the ester decomposition promoter in the obtained films were as shown in Table 1.

In Comparative Examples 1 to 6, by similarly using the Laboplusto-mill (manufactured by Toyo Seiki Co.), the PLA (4032D manufactured by Natureworks Co.) was formed at 210°C into 100 $\mu$m-thick PLA films.

Enzyme;

Protease (product name: Savinase 16.0L, manufactured by Novozymes Co.) was used.

Enzyme adsorption-suppressing compound;

Tris(hydroxymethyl)aminomethane, research grade (manufactured by Wako-Junyaku Kogyo Co.) was used.

Testing the decomposition of the films with the enzyme;

To Tris buffer solutions prepared at concentrations shown in Table 1, each in an amount of 200 ml, the sodium chloride was added in such amounts that the concentrations were 3% and 10% as shown in Table 1, and to each of which 700 $\mu$L of the Savinase enzyme solution was added to prepare decomposition solutions. The PLA films or the PLA films containing the ester decomposition promoter cut into 2 cm x 2 cm (120 mg) were dipped in the decomposition solutions and were shaken at 45°C and at 100 rpm for one week. Thereafter, the films were taken out and were dried at 70°C for 3 hours. The decomposition ratios were found according to the following formula,

$$\text{Decomposition ratio (\%)} = [(\text{initial weight of the film} - \text{weight of the film after decomposed})/\text{initial weight of the film}] \times 100$$

Table 1

|  | PEOx content in film (wt%) | Tris concentration (wt%) | NaCl Concentration (wt%) | Decomposition ratio after one week (%) |
|---|---|---|---|---|
| Example 1 | 5 | 0.05 | 3 | 92.2 |
| Example 2 | 5 | 0.1 | 3 | 100 |
| Example 3 | 5 | 0.05 | 10 | 75.2 |
| Example 4 | 5 | 0.1 | 10 | 100 |
| Example 5 | 5 | 0.01 | 3 | 16.4 |
| Example 6 | 5 | 0.01 | 10 | 11.2 |
| Comp. Ex. 1 | 0 | 0.01 | 3 | 7.6 |
| Comp. Ex. 2 | 0 | 0.05 | 3 | 6.8 |
| Comp. Ex. 3 | 0 | 0.1 | 3 | 4.8 |
| Comp. Ex. 4 | 0 | 0.01 | 10 | 4.1 |
| Comp. Ex. 5 | 0 | 0.05 | 10 | 5.7 |
| Comp. Ex. 6 | 0 | 0.1 | 10 | 4.0 |

**Claims**

1. A method of decomposing an ester resin with an enzyme by making present an ester decomposition promotor, the enzyme and an enzyme adsorption-suppressing compound in an aqueous medium that contains said ester resin.

2. The decomposition method according to claim 1, wherein said ester resin is a polylactic acid.

3. The decomposition method according to claim 1, wherein as said ester decomposition promoter, use is made of an acid-releasing hydrolysable resin that releases an oxalic acid or a glycolic acid upon hydrolysis.

4. The decomposition method according to claim 3, wherein said acid-releasing hydrolysable resin is polyoxalate.

5. The decomposition method according to claim 1, wherein as said enzyme, use is made of at least one selected from protease, lipase and cutinase.

6. The decomposition method according to claim 1, wherein a trishydroxymethylaminomethane is used as said compound for the enzyme adsorption-suppressing compound.

7.  An enzymatically decomposable aqueous dispersion solution including an aqueous medium in which are dispersed an ester resin, the ester decomposition promoter for promoting the hydrolysis of the ester resin, and an enzyme, and in which is, further, contained an enzyme adsorption-suppressing compound.

8.  The dispersion solution according to claim 7, wherein said the enzyme adsorption-suppressing compound is contained in amount of not less than 0.01% by mass relative to said aqueous medium.

9.  The dispersion solution according to claim 7, wherein said enzymatically decomposable aqueous dispersion solution is used for drilling an underground resources.

10. A method of extracting the underground resources from an ore chute formed by drilling, including a step of hydrolyzing an ester resin in water of not lower than 40°C by forcibly introducing the enzymatically decomposable aqueous dispersion solution of claim 9 into the ore chute.

Fig. 1

## NaCl 3%

Fig. 2

## NaCl 10%

## INTERNATIONAL SEARCH REPORT

| International application No. |
|---|
| PCT/JP2015/065143 |

| A. CLASSIFICATION OF SUBJECT MATTER |
|---|
| *C08J11/28*(2006.01)i, *C09K8/12*(2006.01)i, *C12N9/18*(2006.01)i, *E21B43/22* (2006.01)i |
| According to International Patent Classification (IPC) or to both national classification and IPC |

| B. FIELDS SEARCHED |
|---|
| Minimum documentation searched (classification system followed by classification symbols) |
| C08J11/28, C09K8/12, C12N9/18, E21B43/22 |

| Documentation searched other than minimum documentation to the extent that such documents are included in the fields searched |
|---|
| Jitsuyo Shinan Koho        1922–1996   Jitsuyo Shinan Toroku Koho   1996–2015 |
| Kokai Jitsuyo Shinan Koho   1971–2015   Toroku Jitsuyo Shinan Koho   1994–2015 |

| Electronic data base consulted during the international search (name of data base and, where practicable, search terms used) |
|---|
| |

### C. DOCUMENTS CONSIDERED TO BE RELEVANT

| Category* | Citation of document, with indication, where appropriate, of the relevant passages | Relevant to claim No. |
|---|---|---|
| X<br>A | JP 2010-116482 A  (Toyo Seikan Kaisha, Ltd.),<br>27 May 2010 (27.05.2010),<br>claims 1, 5, 6; paragraphs [0001], [0014], [0015], [0020], [0021], [0035]<br>(Family: none) | 1–8<br>9–10 |
| A | JP 2010-138390 A  (Toyo Seikan Kaisha, Ltd.),<br>24 June 2010 (24.06.2010),<br>claims 1, 3, 8; paragraphs [0025], [0031]<br>& US 2011/0201069 A1    & EP 2348122 A1<br>& CN 102264912 A | 1–10 |
| A | JP 2010-116481 A  (Toyo Seikan Kaisha, Ltd.),<br>27 May 2010 (27.05.2010),<br>claims 1, 8; paragraphs [0024], [0026]<br>(Family: none) | 1–10 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 July 2015 (28.07.15) | 04 August 2015 (04.08.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

| International application No. |
| --- |
| PCT/JP2015/065143 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2011-006615 A (Toyo Seikan Kaisha, Ltd.), 13 January 2011 (13.01.2011), claims 1, 5; paragraphs [0001], [0047] (Family: none) | 1-10 |
| A | WO 2012/121294 A1 (Kureha Corp.), 13 September 2012 (13.09.2012), paragraphs [0001] to [0003], [0025] (Family: none) | 1-10 |
| A | WO 2012/050187 A1 (Kureha Corp.), 19 April 2012 (19.04.2012), paragraphs [0001], [0002], [0011] & US 2013/0252854 A1   & CN 103154182 A | 1-10 |
| P,X | WO 2014/092146 A1 (Toyo Seikan Kaisha, Ltd.), 19 June 2014 (19.06.2014), paragraphs [0021], [0029], [0037], [0041]; claims & AU 2013358061 A    & CA 2892496 A | 1-10 |
| P,X | JP 2014-177618 A (Toyo Seikan Kaisha, Ltd.), 25 September 2014 (25.09.2014), claims; paragraphs [0001], [0016], [0018], [0027], [0034] & AU 2013358061 A    & CA 2892496 A | 1-10 |

Form PCT/ISA/210 (continuation of second sheet) (July 2009)

**EP 3 150 659 A1**

**REFERENCES CITED IN THE DESCRIPTION**

**Patent documents cited in the description**

- US P7833950 A
- WO 2012050187 A
- WO 2014092146 A